# EUROPEAN PATENT APPLICATION

(11) **EP 3 624 135 A1**
(43) Date of publication of application: **18.03.2020**
(21) Application number: 18194022.2
(22) Date of filing: 12.09.2018
(51) Int. Cl.: G16H 50/30, G16H 50/70, G16H 15/00

(54) **TRIGGERING AN ALERT FOR A SUBJECT**

(71) Applicant: Koninklijke Philips N.V., 5656 AG Eindhoven (NL)
(72) Inventor: ZHU, Yajing, 5656 AE Eindhoven (NL); ZHU, Qin, 5656 AE Eindhoven (NL); JIANG, JIE, 5656 AE Eindhoven (NL); YIN, Bin, 5656 AE Eindhoven (NL); HUO, Yong, 5656 AE Eindhoven (NL)
(74) Representative: de Haan, Poul Erik

(57) **Abstract**

A method and system for triggering an alert for a subject. A prediction model is applied to current health data to predict a quality of life score of the subject. A determination is made as to whether the quality of life score meets one or more predetermined criteria, and thereby whether to trigger an alert. An alert is triggered if the quality of life score meets (at least one of) the one or more predetermined criteria. The prediction model is generated by applying a machine learning algorithm to a dataset formed of data records, each data record associating at least (historical) health data with (historical) quality of life scores.

## Description

### FIELD OF THE INVENTION

The invention relates to a method or system for triggering an alert for a subject, and in particular, for triggering an alert when a measure of the subject's health falls below an acceptable level.

### BACKGROUND OF THE INVENTION

In the medical profession, there is a strong desire to monitor or track a patient's health. This is especially important after a patient has been discharged from hospital or for long-term (chronic) conditions. This helps prevent or recognize possible deterioration of a patient when not in a clinical environment, i.e. where there are no nearby clinical staff or pieces of medical equipment. In such scenarios, it is important to quickly recognize when a patient's health has fallen or will fall below an acceptable level, so that there is sufficient time for intervention before further or irreversible deterioration occurs.

One way to measure a patient's health level is to obtain a Quality of Life (QOL) score of the patient. The QOL score is a known measure of an individual's perceived well-being (i.e. his/her 'health status'), and is regularly used in post-hospital management to monitor how effective a health treatment has been as well as the patient's ongoing condition.

Conventionally, to obtain a QOL score, an individual takes a quality of life questionnaire, for example, during a follow-up visit to/from a doctor. However, obtaining a QOL score from a patient is difficult, especially following the patient's release from a hospital setting, due to a high cost of health care and poor patient compliance.

There is therefore a desire to provide an improved method of determining a perceived health status of a patient, which has a reduced reliance on a patient performing a questionnaire or other Quality of Life assessment.

### SUMMARY OF THE INVENTION

The invention is defined by the claims.

According to examples in accordance with an aspect of the invention, there is provided a method of triggering an alert for a subject, the method comprising: obtaining current health data of the subject; obtaining a prediction model for predicting a quality of life, QOL, score of the subject based on the current health data of the subject, wherein the prediction model is generated by performing a machine learning algorithm using a dataset formed of a plurality of data records, wherein each data record of the dataset comprises health data and an associated QOL score; predicting, using the prediction model, a QOL score of the subject based on the current health data of the subject; determining whether the predicted QOL score meets at least one predetermined criterion, and in response to the predicted QOL score meeting the at least one predetermined criterion, triggering an alert.

Thus, a quality of life score for a subject is obtained without the need for a subject to perform or undertake a dedicated quality of life assessment. In particular, health data of a subject is processed, using a prediction model, to predict a quality of life score of the subject. The predicted quality of life score is used to determine whether to trigger an alert.

It is important to use a quality of life score (e.g. rather than any other health metric) for triggering the alert because a subject's perceived well-being (QOL score) has a significant impact on their health, happiness and treatment urgency. A deterioration in quality of life results in reduced happiness and subject satisfaction, and can lead to a reduced lifespan. A quality of life score is therefore considered an important indicator of a need for intervention.

In order to predict a quality of life of the subject using the health data, a prediction model is obtained. In particular, the prediction model has been generated using historical health data, e.g. of other subjects and/or the subject under investigation, and associated historical quality of life scores. A machine learning algorithm is applied to this historical health data to identify (causal) links between values of the historical health data and the historical quality of life scores. These links can be used to generate a prediction model for predicting a quality of life score of the subject based on at least current health data of the subject. Examples of suitable machine learning algorithms include a regression algorithm, a random forest algorithm, a support vector machine methodology and so on.

Thus, the present invention proposes to use a prediction model, formed by linking historical health data records with a quality of life score, to predict a quality of life score for a (new) subject using a current health data record of the subject.

This reduces a burden on the subject or clinician in performing a quality of life assessment, as such an assessment can be done automatically. It also ensures that potentially relevant clinical events are not overlooked (e.g. by a clinician or subject) in the assessment of their quality of life scores. Such a method no longer needs to rely on potentially expensive quality of life assessments or subject compliance with such quality of life assessments.

Furthermore, an alert can be automatically triggered based on changes in the subject's condition that affect their predicted quality of life. In other words, current health data is processed to predict a quality of life score, which can trigger an alert. This advantageously decreases a likelihood that a deterioration in a subject's health condition will be missed, allowing for suitable timely intervention.

The proposed method also avoids a need for complex and time-consuming analysis of subject health data to assess a condition of a subject. Thus, by performing a quality of life prediction using historical data, an accurate representation of the subject's perceived state of well-being can be ascertained with a high degree of certainty without requiring lengthy and costly consultations with the subject.

The quality of life score may represent a quality of life over a predetermined period of time (e.g. a week, a fortnight or a month). Accordingly, the current health data of the subject may represent health data of a subject obtained over the predetermined period of time.

The step of obtaining the prediction model comprises generating a prediction model by performing a machine learning algorithm on the dataset, wherein each data record of the dataset comprises health data and an associated QOL score. Thus, each element/entry of a data set may comprises health data and a QOL score.

In other examples, a stored prediction model is used, wherein the stored prediction model has previously been generated based on a dataset, each element/entry of which comprises health data and an associated QOL score.

The step of obtaining the prediction model may comprise: generating health data for each data record of the dataset by performing a natural language processing procedure on health notes of a subject associated with a QOL score.

Thus, there may be a plurality of health notes (each associated with a QOL score). Each of the plurality of health notes may be processed, using a natural language processing procedure, to generate respective health data for a data record of the dataset - which data record also comprises the QOL score associated with the health note.

Thus, a health note (i.e. text describing health information) can be converted into health data (recognizable by a computer, such as numerical information). A health note is usually unstructured text, a format which cannot usually be recognized by a computing system. Natural language processing (NLP) can therefore be used to transfer a health note into health data that is in a format that can be easily processed by computer programs.

Using a natural language processing procedure to extract health data reduces a burden of inputting information into a database for a subject or clinician. This allows increased compatibility of the method with existing historic data information; as such historic data information could be stored in various different formats, which could be processed to extract relevant health data using natural language processing.

Health notes are a generally accepted and intuitive method of recording health data of a subject. In particular, a subject/clinician does not need to input information in a particular format or structure in order to have their health data recorded. This reduces a burden on the subject or clinician, and increases a likelihood that they will provide their health information. By increasing this likelihood, it is less likely that deterioration of the subject will be missed or overlooked.

The method may comprise obtaining demographic information of the subject, wherein the step of obtaining the prediction model comprises obtaining the prediction model based on at least the demographic information of the subject.

By obtaining the prediction model based on demographic information (e.g. age, gender and so on), the prediction model may more accurately model the quality of life of the subject. In this way, the quality of life score will be more accurate, thereby reducing a number of false alerts and improving an accuracy of the alerts.

Obtaining the prediction model based on the demographic information may comprise, for example, filtering the plurality of health data and QOL scores of other subjects to those subjects having similar demographic information to the subject under investigation, and obtaining the prediction model from only those subjects. Other embodiments will be apparent to the skilled person (e.g. weighting other subject data based on their demographic similarity to the subject under investigation).

In other examples, the prediction model is further adapted to receive, as input, demographic information, and generate a QOL score based on the demographic information and the current health data. Thus, the step of predicting, using the prediction model, may comprise using the prediction model to predict a QOL score using current health data and demographic information of the subject.

The method may comprise obtaining diagnosis and/or recommended treatment information of the subject, wherein the step of obtaining the prediction model comprises obtaining the prediction model based on at least the diagnosis and/or recommended treatment information of the subject.

Thus, clinician-sourced information (diagnoses or recommended treatment plans) may be obtained. The clinician-sourced information is used to obtain the prediction model for predicting the quality of life score of the subject.

This improves an accuracy and reliability of the prediction model, thereby ensuring the predicted quality of life score is more accurate, reducing a number of false alerts.

In other examples, the prediction model is further adapted to receive, as input, diagnosis and/or recommended treatment information of the subject, and generate a QOL score based on the diagnosis and/or recommended treatment information and the current health data. Thus, the step of predicting, using the prediction model, may comprise using the prediction model to predict a QOL score using current health data and diagnosis and/or recommended treatment information of the subject.

Of course, the prediction model may be adapted to process any information associated with the subject when predicting the QOL score. For example, step of predicting, using the prediction model, may comprise using the prediction model to predict a QOL score using current health data, demographic information and diagnosis and/or recommended treatment information of the subject.

In embodiments, the step of determining whether the predicted QOL score meets at least one predetermined criterion comprises determining whether the predicted QOL score is less than a first predetermined value.

In this way, an alert can be triggered when the subject's quality of life has deteriorated below an acceptable or warning value (the first predetermined value). This helps ensure that there is timely intervention of clinical/medical personnel to prevent further deterioration of the subject.

The first predetermined value may therefore represent a value associated with a minimum acceptable quality of life level, a quality of life level approaching a minimum acceptable level or a minimum desired quality of life level (e.g. for a particular clinical setting or healthcare provider, or for the subject themselves).

In at least one embodiment, the step of determining whether the predicted QOL score meets the at least one predetermined criterion comprises determining whether a difference between the predicted QOL score and a previous QOL score of the subject is greater than a second predetermined value.

In this way, an alert may be triggered if there is a sudden change in a subject's quality of life. This may be indicative of a sudden deterioration in subject condition, and indicative of an imminent need for medical intervention. Thus, triggering an alert when there is a sudden change in a quality of life score can prevent their pathological condition from (irreversibly) declining.

The previous quality of life score may be obtained, for example, from a dataset storing previous QOL scores of the subject. The previous quality of life score is preferably a most immediately previous quality of life, or may be an average of a predetermined number of previous quality of life scores. The previous quality of life score may be a previously predicted quality of life score (e.g. from a previous iteration of the method) or a quality of life score obtained from the subject, e.g. via an assessment or questionnaire.

The previous quality of life may represent a quality of life in a previous week, fortnight (two-week period) or month.

Optionally, the current health data comprises at least one measure of a sign, symptom, lifestyle and/or emotion of the subject. Lifestyle measures may comprise, for example, measures concerning a subject's sleep, exercise, diet, work, leisure time and so on.

The current health data may therefore comprise any measure of information that may affect or indicate a condition of a subject. For example, reduced quality of exercise or reduced quality of sleep (lifestyle measures) may indicate that a condition of a subject has deteriorated.

By way of example, the current health data may comprise information on the subject's symptoms (e.g. headaches, chest distress etc.), sleeping habits (e.g. number of occasions of insomnia, nightmares, easily awoken etc.), exercise measures (e.g. number of times gone swimming, walking, doing housework etc.), dietary information (e.g. number of occasions of losing appetite, alcohol drinking etc.), or emotional status (e.g. happiness value, mood scale value, number of times subject has felt depressed/anxious etc.).

According to an embodiment, the current health data comprises one or more categories of current health information of the subject, and the method further comprises: obtaining previous health data comprising a respective one or more categories of previous health information of the subject; comparing each category of the current heath data to a respective category of the previous heath data to obtain a respective one or more similarity values indicative of a similarity between a category of the current health data and the respective category of the previous health data; and in response to at least one of the one or more similarity values being lower than a predetermined threshold value, triggering an alert.

Thus, a sudden change in subject characteristics or information will trigger an alert. Sudden changes in health-related subject characteristics, such as symptoms, may be indicative of a sudden deterioration in subject condition and therefore may indicate an imminent need for medical intervention. Thus, triggering an alert when there is a sudden change in a subject's health data can prevent their pathological condition from (irreversibly) declining.

Optionally, each category of the current health data comprises a respective first time series representative of a change in current health information of the subject over time; each category of the previous health data comprises a respective second time series representative of a change in previous health information of the subject over time, and wherein the step of comparing each category of the current health data to a respective category of the previous health data comprises comparing the first time series, of each category of the current health data, to the second time series, of a respective category of the previous health data, to obtain the similarity value.

Thus, a portion of the current health data of the subject over a first period of time may be compared to a corresponding portion of previous health data of the subject over a second, earlier period of time. Preferably, the two periods of time are identical in length.

Comparing a time series of health data means that anomalous spikes (e.g. to an unexpected change in lifestyle, such as a party) can be averaged out, and reduces a false positive rate of triggering an alert.

In some embodiments, the step of comparing the first time series to the second time series comprises calculating a time series correlation between the first and second time series.

In embodiments, the step of obtaining current health data of the subject comprises performing a natural language processing procedure on health notes of the subject to obtain the current health data.

In exemplary embodiments, the current health data of the subject comprises health information gathered over the course of a time period of a predetermined length, and the health data contained in data record of the dataset comprises health information gathered over the course of a time period of the same predetermined length. The predetermined time interval may be a month, but may be of other lengths of time, such as a day, week, fortnight (two-week period) or year.

There is also proposed a computer program comprising code means for implementing any previously described method when said program is run on a computer.

There is also proposed a system for triggering an alert for a subject, the system comprising: an obtaining unit adapted to: obtain current health data of the subject; and obtain a prediction model for predicting a quality of life, QOL, score of the subject based on the current health data of the subject, wherein the prediction model is generated by performing a machine learning algorithm using a dataset formed of a plurality of data records, wherein each data record of the dataset comprises health data and an associated QOL score; a predicting unit adapted to predict, using the prediction model, a QOL score of the subject based on the current health data of the subject; a determining unit adapted to determine whether the predicted QOL score meets at least one predetermined criterion, and a triggering unit adapted to, in response to the predicted QOL score meeting the at least one predetermined criterion, trigger an alert.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a method according to an embodiment;
Figures 2 and 3 illustrate different embodiments for a step of determining whether a QOL score meets at least one predetermined criterion;
Figures 4 and 5 illustrate different embodiments of generating a prediction model or models;
Figure 6 illustrate a method according to another embodiment of the invention;
Figure 7 illustrates a framework for determining whether to trigger an alert; and
Figure 8 illustrates a system according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a method or system of triggering an alert for a subject. A prediction model is applied to current health data to predict a quality of life score of the subject. A determination is made as to whether the quality of life score meets one or more predetermined criteria, and thereby whether to trigger an alert. An alert is triggered if the quality of life score meets (at least one of) the one or more predetermined criteria. The prediction model is generated by applying a machine learning algorithm to a dataset formed of data records, each data record associating at least (historical) health data with (historical) quality of life scores.

Embodiments are at least partly based on the realization that a measure of a subject's quality of life is an important tool for assessing a condition of the subject, but that assessing a quality of life is typically a complex and time-consuming task. The inventors have recognized that a measure of quality of life can be predicted automatically using current health data of the subject. In particular, a prediction model may be generated from a dataset that correlates (historical) health data to QOL scores, which can be used to predict a current quality of life score.

Illustrative embodiments may, for example, be employed in post discharge subject monitoring solutions or for monitoring a condition of the subject having a long-term chronic disease.

In the field of medicine, the term 'subject' is commonly used to refer to a patient, individual or person, such as one in a hospital (i.e. a patient) or discharged from a clinical setting (i.e. so that they are no longer considered a patient).

The term 'quality of life' is a measure of the subject or individual's perceived well-being, e.g. of day-to-day health. Thus, in the context of the present invention, quality of life refers to a health-related quality of life. By way of further example, a health-related quality of life (QOL) score may be a measure of how the individual's well-being may be affected over time by a disease, disability, or disorder.

The term 'health data' refers to information on a subject's signs/symptoms (e.g. vital signs or occurrences of medical conditions such as headaches/chest distress), or other lifestyle/emotional measures which can affect or indicate changes to the subject's health, such as sleep information, exercise information, diet information or emotional (e.g. mood) information. Thus, health data indicates information relevant to a subject's health (and is non-demographic information). In particular, health data may comprise one or more measures of a subject's health (e.g. occurrence of symptoms) or health-influencing factors (such as sleep, diet, exercise or emotion information).

Preferably, health data indicates information relevant to a subject's health that has been acquired over a predetermined length of time (e.g. a day, a week or month). Thus, for example, health data may indicate a number or frequency of heart problems over the course of a month. In another example, health data may indicate a number of times a subject has gone swimming over the course of a month.

In the context of the present invention, the term 'prediction model' refers to any suitable methodology, function or equation that receives, as input, at least health data of a subject and outputs a predicted QOL score for the subject based on that health data.

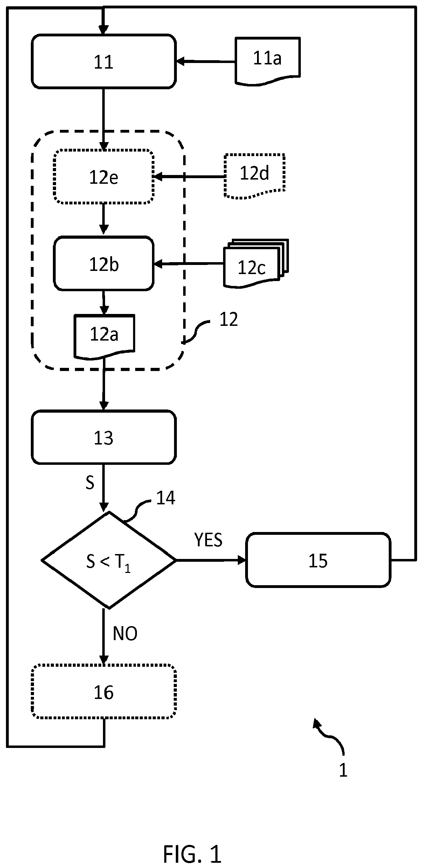

Figure 1 illustrates a flow diagram for a computer-implemented method 1, for triggering an alert for subject, according to an embodiment of the invention.

The method 1 begins with step 11, which comprises obtaining current health data 11a of the subject. This may comprise, for example, obtaining current health data 11a from a storage device.

In some other embodiments, step 11 comprises performing a natural language processing procedure on health notes of the subject to obtain the current health data 11a. This reduces a burden on the subject/clinician to provide health information in a computer-compatible format, and increases a flexibility of the method to incorporate existing subject notes (a typical method of recording subject information) thereby increasing a potential market for the method.

Current health data may represent the most recently available (i.e. in time) health-related information of an individual or subject, or may instead represent health information gathered over previous period of time.

The method 1 also comprises step 12, which comprises obtaining a prediction model 12a for predicting a quality of life (QOL) score of the subject (based on the current health data 11a). Suitable methods of generating or otherwise obtaining a prediction model will be described later. Steps 11 and 12 may be performed in any order, or in parallel to one another.

In subsequent step 13, a QOL score S of the subject is predicted using the prediction model, based on the current health data of the subject. Put another way, step 13 comprises applying a prediction model to the current health data to predict a quality of life score. Thus, the current health data is used as an input to a prediction model that processes the current health data to output a predicted QOL score of the subject.

A QOL score S may be a value or numerical measure representative of the subject's perceived quality of life. For example, it may be a number between 0 and 100, where 0 represents a minimum possible/conceivable quality of life for the subject and 100 represents a maximum possible quality of life for the subject. In other examples, the quality of life score may range between 0 and 1. The quality of life score range may be defined in accordance with standard policies or medical practices, or by a caregiver/therapist for the subject.

Step 14 comprises determining whether the predicted quality of life (QOL) score S meets at least one predetermined criteria.

In the illustrated (simple) example, step 14 comprises determining whether the quality of life (QOL) score S is below a first predetermined value T₁. Thus, the at least one predetermined criteria may comprise a criterion that the predicted QOL score is less than a first predetermined value T₁. The first predetermined value may represent a minimum recommended or subject-desired value for the QOL score, or may represent a QOL score indicative of a poor subject condition. Where the QOL score is a measure between 0 and 100, the first predetermined value may, for example, be 60. Thus, the first predetermined value may be 60% of the maximum possible quality of life value.

In response to the method determining, in step 14, that the predicted QOL score meets the at least one predetermined criterion, an alert is triggered or generated in step 15. This may comprise, for example outputting an audio/visual signal indicative of the QOL score meeting the at least one predetermined criterion.

Optionally, in response to the method determining, in step 14, that the predicted QOL score does not meet the at least one predetermined criteria, an indicator may be provided in step 16 that the QOL score meets acceptable criteria (e.g. falls within allowable bands). Thus, no alert is triggered if the predicted QOL score does not meet the at least one criteria for which an alert would be triggered.

The proposed method enables an alert to be triggered when a subject's quality of life meets one or more predetermined criteria which is representative of a poor subject condition, enabling subject deterioration to be detected promptly and automatically. The proposed method also reduces a need to perform potentially time-consuming or expensive assessments of a subject's quality of life, as this may be automatically predicted via a prediction model.

In some embodiments, step 12 comprises obtaining a single prediction model (e.g. that has been previously used for the subject). This provides a simple implementation of the method.

However, in other embodiments and as illustrated in Figure 1, the step 12 may instead comprise a sub-step 12b of selecting one of a plurality of potential prediction models 12c for use as the prediction model 12a for predicting the QOL score.

In at least one example, each potential prediction model 12c is associated with different demographic information, and the sub-step 12b may comprise obtaining the prediction model based on the demographic information 12d of the subject. Byway of example, sub-step 12b may comprise identifying the potential prediction model associated with demographic information closest to the demographic information of the subject, and selecting this identified potential prediction model as the prediction model.

In this way, the step of obtaining the prediction model 12 may comprise a sub-step 12b of obtaining the prediction model based on at least the demographic information 12d of the subject. Moreover, the step 12 may comprise a sub-step 12e of obtaining demographic information 12d of the subject.

In other examples, each potential prediction model 12c is associated with different possible diagnostic or treatment information of a subject, and the sub-step 12b may comprise obtaining the prediction model (from the plurality of prediction models) based on diagnostic and/or treatment information of the subject.

In this way, the step of obtaining the prediction model 12 may comprise a sub-step 12b of obtaining the prediction model based on at least the diagnosis and/or treatment information 12d of the subject.

The term "treatment information" refers to information about the recommended treatment of the subject, such as recommended medications/dosages, lifestyle changes, therapeutic sessions and so on. The term "diagnosis information" refers to information about the diagnosis or medical history of the subject, such as a clinical diagnosis, identification of potential illness/problem affecting the subject, history of asthma, diabetes etc., and so on.

Of course, each of the potential prediction models 12c may be associated with both demographic information and diagnostic and/or treatment information for a subject, and a prediction model may be selected based on the appropriate characteristics of the subject (under investigation).

Thus, different (potential) prediction models may be associated with different potential characteristics for a subject, and the most appropriate prediction model (i.e. the prediction model associated with characteristics of a subject closest to the characteristics of the current subject) may be selected for predicting the QOL score.

In some examples, a prediction model is associated with a particular cluster of historical health data instances. The step 12b may comprise assigning the health data of the subject to a cluster, and selecting the associated prediction model associated with that cluster.

This means that a selected prediction model will be more appropriate for the condition or characteristics of the subject under investigation, increasing an accuracy of predicting the QOL score.

In other examples, a prediction model may be able to receive, as input, the demographic information and/or diagnostic information and/or treatment information in order to predict the QOL score. Thus, the step 12 of obtaining a prediction model may comprise obtaining a single prediction model (capable of processing such information of a subject).

In this way, the step 13 may comprise predicting a QOL score of the subject using the prediction model, based on the current health data of the subject and one or more of: demographic information, diagnostic information and/or treatment information. In particular, step 13 may comprise inputting such information to a prediction model and outputting, from the prediction model, a predicted QOL score.

The method 1 may be iteratively repeated (or multiple instances of method 1 performed in parallel) for different health data of a subject. For example, a subject may be associated with multiple instances of health data, each of which may be consecutively/concurrently processed to predict a respective QOL score for each instance of health data.

Each instance of health data may represent a different period over which health data has been gathered. Thus, a first instance (processable by the method 1) may represent a first period of time, and a second instance (also processable by the method 1) may represent a subsequent second period of time.

For example, health data of a subject gathered over the course of a year may be divided into health data for each month. A QOL score, for each month, may be generated by iteratively repeating the method (with current health data changing with each iteration) for each month.

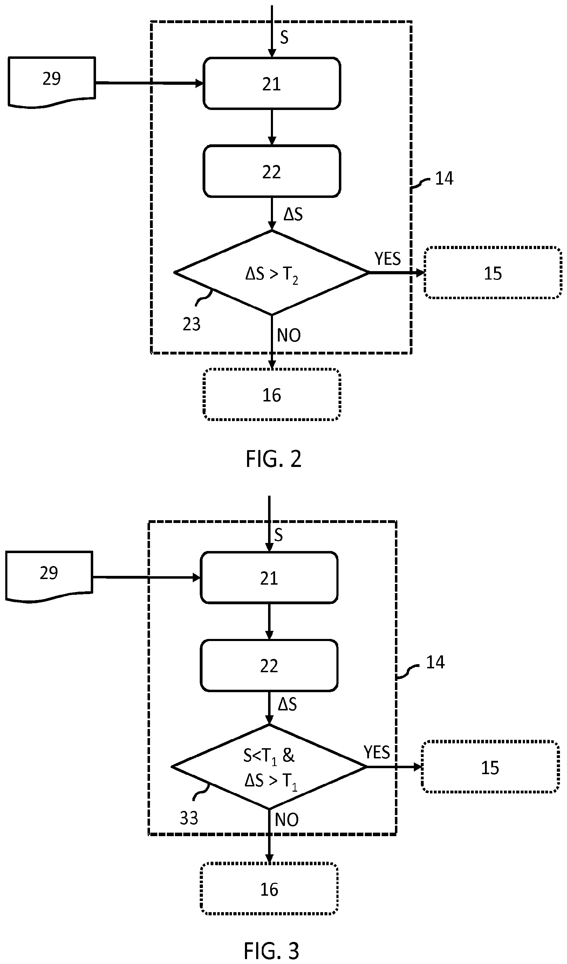

Figures 2 and 3 illustrate alternative embodiments for step 14 for use in the method 1 of Figure 1.

Referring first to Figure 2, step 14 comprises a sub-step 21 of obtaining a previous QOL score 29 of the subject. The previous QOL score 29 may be a QOL score obtained during a previous iteration of method 1, or may be a stored QOL score from an earlier QOL assessment. In some examples, the previous QOL score is instead an average (e.g. over a previous year, month of week) of a plurality of previous QOL scores.

Step 14 also comprises a sub-step 22 of determining a difference between the previous QOL score 29 and the predicted QOL score (obtained in previous step 13). The determined difference ΔS may be a numerical difference (e.g. subtracting the predicted QOL score from the previous QOL score) or a percentage difference (e.g. determining a percentage change from the previous QOL score to the predicted QOL score).

Step 14 also comprises a sub-step 23 of determining whether the difference ΔS between the previous QOL score 29 and the predicted QOL score is greater than a second predetermined value T₂. Thus, sub-step 23 determines whether the predicted QOL score is worse (i.e. less) than the previous QOL score by at least a second predetermined value. Thus, a predetermined criterion of step 14 may be that a difference between a previous QOL score and the predicted QOL score must be greater than a second predetermined value T₂.

In response to the sub-step 23 determining that the difference is greater than the second predetermined value T₂, the method 1 moves to step 15 of triggering an alert. Otherwise, no alert is triggered (e.g. moves to optional step 16).

Where the difference is a percentage difference, the second predetermined value may be 50%, i.e. the alert may be triggered when the predicted QOL value is 50% or more lower than the previous QOL value.

In this way, a sudden change in quality of life score will trigger an alert, reducing a likelihood that a deterioration in subject condition will be missed and thereby ensuring that timely intervention is provided to improve subject outcome.

In Figure 3, the step 14 comprises two predetermined criteria, both of which must be met for the method to move to step 15. This maybe alternatively viewed as a single criteria, formed of two parts or sub-criteria.

In particular, step 14 may, in place of sub-step 23, comprise a sub-step 33 of determining whether the predicted QOL score (S) is less than a first predetermined value T₁ and the difference (ΔS) between the predicted QOL score and a previous QOL score is greater than a second predetermined value T₂.

Thus, a predetermined criterion of step 14 may be that the difference ΔS must be greater than a second predetermined value and that the predicted QOL score is less that a first predetermined value.

This prevents mild variations in the predicted QOL score (which could dip below the first predetermined value) from generating an unnecessary alert, providing a more appropriate assessment.

In addition to the above described examples, it will be appreciated that step 14 may comprise determining whether the predicted QOL score meets any one of a plurality of criterions.

By way of example, step 14 may comprise determining whether the quality of life score meets either criteria of the predicted quality of life score S being less than a first predetermined value or the difference ΔS being greater than a second predetermined value-any moving to step 15 in the event of either criteria being met.

It will be appreciated that step 14 may comprise determining whether any one of a plurality of criteria or combination of sub-criteria are met, of which at least one criterion is based on the predicted QOL score. Thus, a single criterion may comprise a plurality of sub-criteria, all of which must be met for the criterion to be considered met. Thus, an alert may be triggered based using any "and/or" combination of possible criteria.

For example, step 14 may comprise determining whether: the difference ΔS is greater than a second predetermined value and the predicted quality of life score is less than a first predetermined value; or the quality of life score is less than third predetermined value.

In such an embodiment, the third predetermined value is preferably less than the first predetermined value. Such an example will prevent any minor variations in the predicted QOL score (e.g. dipping below the first predetermined value) from triggering an alert, whilst still allowing an alert to be triggered when the predicted QOL score is not sufficiently high (i.e. when below the third predetermined value). This prevents a slowly deteriorating subject from being overlooked.

Where the QOL score is a measure between 0 and 100, a difference between the first and third predetermined values may be 10/15/20 (i.e. the third predetermined value may be 10/15/20 less than the first predetermined value). In other examples, the third predetermined value may be 10%, 15% or 20% less than the first predetermined value.

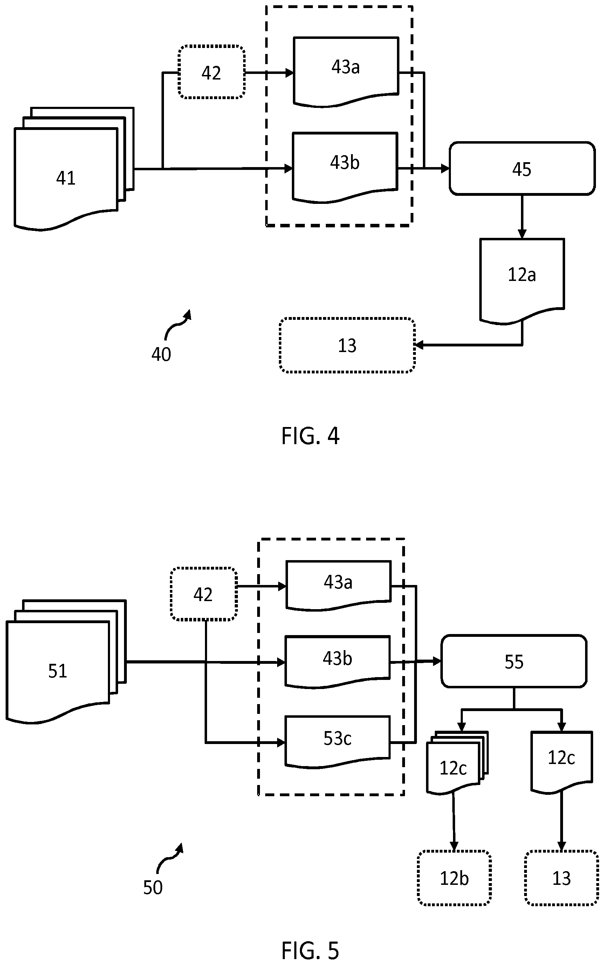

Figure 4 illustrates a framework 40 for a method of generating a prediction model 12a according to an embodiment. The method 1 may comprise such a method of generating a prediction model (e.g. in step 12).

A dataset 41 comprising historical data (e.g. of a plurality of subjects) is used to generate the prediction model. The dataset comprises a plurality of entries or data records, each data record comprising health data 43a of a (historical) subject and an associated QOL score 43b. Thus, each data record associates at least (historical) health data with (historical) quality of life scores The QOL score may have been obtained, for example, using an existing QOL assessment technique.

The health data contained by a single data record may represent at least one measure of a health (or health-affecting characteristic) of a subject over a predetermined period of time (e.g. a month). Thus, in an example, the health data of a single record may indicate a number of headaches reported by a subject over the course of a month.

The framework comprises a step 45 of performing a machine learning algorithm on the dataset to generate the prediction model 12a. The machine learning algorithm identifies links or relationships between the health data 43a and the associated QOL score 43b by assessing the dataset. In this way, the machine learning algorithm can generate a prediction model for predicting a QOL score based on health data.

By way of example, step 45 may comprise building a prediction model using a regression method such as logistic regression, in which QOL score is a dependent value and the remaining parameters (i.e. parameters of the health data) are independent values. Other suitable machine learning algorithms include a random forest algorithm or a support vector machine methodology, although other methods will be apparent to the skilled person.

In this way, the machine learning algorithm, by assessing the dataset, generates a prediction model that takes account of the relationship between health data and a QOL score, such as a weighted function with different aspects of health data 43a as (independent) variables and the QOL score as an output (dependent) variable.

In some embodiments, each data record of the dataset 41 comprises health notes of a subject and an associated QOL score. The framework may comprise a step 42 of applying a natural language processing (NLP) procedure on the health notes of each data record, to thereby generate health data for each data record of the dataset.

For example, step 42 may comprise applying a NLP procedure to extract data from the health notes, and transform the extracted data into digital data that can be recognized by a machine learning algorithm. By way of example, the NLP procedure may comprise identifying at least five categories of health information (including symptom information, sleeping information, exercise information, diet information and mood conditions) associated with the subject.

The entries of the dataset 41 may be associated solely with the subject under investigation, so that each data record comprises historical health data and a historical QOL score of the subject. In other examples, the entries of the dataset are associated with other subjects (e.g. from a databank of health notes), so that each data record comprises health data and an associated QOL score on other subjects. Of course, in yet other embodiments, the entries of the dataset are associated with a combination of both historical data records of the subject and historical data records of other subjects.

In one example, the dataset comprises at least 5000 data records (e.g. representing at least 5000 people or periods for which health data and a QOL score have been collected), each data record comprising respective health data 43a and an associated QOL score.

Each data record preferably comprises or is associated with health data (or health notes) acquired over a predetermined length of time (e.g. a month or a week) for a subject and a QOL score associated with that period of time for the subject.

Figure 5 illustrates a framework 50 for a method of generating a prediction model 12a, or a plurality of prediction models 12c, according to another embodiment. The method 1 may comprise such a method generating a prediction model.

The framework 50 differs from the earlier framework 40 in that each data record of the dataset 51 further comprises additional, subject-specific information 52c, such as demographic information and/or diagnosis information and/or recommended treatment information. Thus, each data record comprises information on the subject that has provided the heath data of that record and is associated with a particular QOL score.

In one example, step 55 comprises generating a single prediction model 12c that can be used to predict a QOL score based on health data 43a and/or the additional information 43c of a subject.

In particular, step 55 may comprise performing a machine learning algorithm on the dataset 51 to generate the prediction model 12a. The machine learning algorithm identifies links or relationships between the associated QOL score and the combination of the health data 43a and the additional data 53c. The machine learning algorithm, by assessing the dataset, generates a prediction model that takes account of these relationships, such as a weighted function with different health data 43a or additional data 53c measures as variables. In this way, the machine learning algorithm can generate a prediction model for predicting a QOL score based on health data.

In particular, step 45 may comprise building a prediction model using a regression method such as logistic regression, in which the QOL score is a dependent value and the remaining parameters (i.e. parameters/measures of the health data 43a and/or additional information 53c) are independent values. This may generate a function that receives, as input, measures of the health data and additional data to provide an output of the QOL score.

Thus, health data, demographic information, diagnosis information and/or treatment information may be independent variables or input variables of a single prediction model. This means that a single prediction model may be used for any subject, i.e. independently of the characteristics of the subject. This increases a simplicity of predicting a QOL score (according got method 1) and lowers a storage complexity/cost of the prediction model.

In an alternative embodiment, a plurality 12c of different prediction models may be generated in step 45. Each prediction model of the plurality may be associated with different groups or clusters of data records associated with similar subject characteristics.

By way of example, step 45 may comprise clustering the data records of the dataset 51 based upon the additional information of the data records. Thus, for example, data records having similar demographic information may be assigned to a same cluster, and data records having dissimilar demographic information may be assigned to different clusters. A respective prediction model may thereby be generated (using a previously described machine learning algorithm approach) for each cluster of data records. In other words, for a cluster of data records, a machine learning algorithm may identify a relationship between health data and QOL scores to generate a prediction model.

This allows for a divide-and-conquer approach to generating prediction models, allows for reduced complexity of generating prediction models (as fewer variables need to be identified. Such a method also allows a plurality of prediction models to be generated in parallel for a large number of subjects, thereby reducing a processing time.

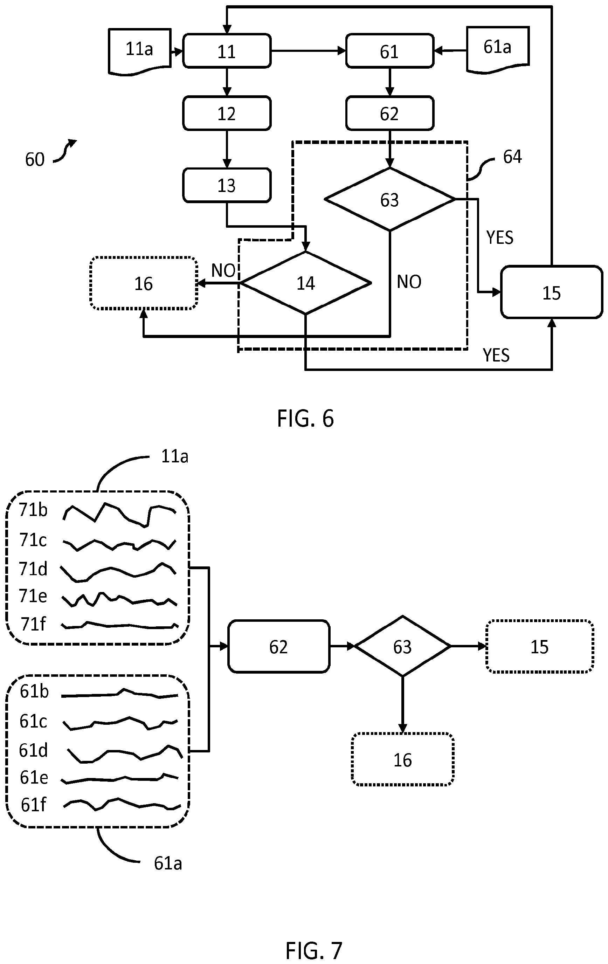

Figure 6 illustrates a computer-implemented method 60 according to an embodiment of the invention.

The method 60 differs from previously described methods by further comprising additional steps 61, 62 and 63. These additional steps allow the integration of previous health data of the subject into the determination of whether or not to generate an alert (i.e. in step 15).

Step 61 comprises obtaining previous health data 61a of the subject. The previous health data is health data that has been gathered prior to the current health data 11a of the subject. For example, where current health data represents health information gathered over the course of a month, the previous health data may represent health information gathered over the course of an immediately preceding month.

The current health data 11a may represent information gathered over a predetermined period of time, and the previous health data 61a may represent information gathered over a period of time (equal in length to that of the current health data) immediately preceding the period over which the current health data was gathered.

The predetermined period of time may, for example, be a week, fortnight (two-week period), month or year. Of course, there may be minor variations in the length of time associated with the current and previous health data, e.g. to account for different lengths of months or years.

Step 62 comprises comparing the previous health data to the current health data in order to generate at least one similarity value indicative of a similarity between the current health data and the previous health data. This may be performed by determining a correlation between the current and previous health data. Generally, the greater the similarity score, the greater a similarity between the current health data and the previous health data.

Alternatively, step 62 may comprise determining a difference score between the current health data and the previous health data (indicative of a difference between the current health data and the previous health data).

Step 63 comprises determining whether the similarity value meets a predetermined criterion. In particular, step 63 may comprise determining whether the similarity value is less than a predetermined threshold value. For example, where the similarity value is a value between 0 and 1, the predetermined threshold value may be in the region of 0.4-0.8, for example between 0.5 and 0.7, such as 0.6.

In response to the determination of step 63 being positive, the method moves to step 15 and generates an alert. In response to the determination of step 63 being negative, the method may move to the step 16 in which no alert is generated, and an indicator is provided that the current health data is similar to previous health data.

Step 63 may be modified accordingly if, in step 62, a difference value is calculated instead of a similarity value. For example, step 63 may instead comprise determining whether a difference value is greater than a predetermined threshold value.

Steps 14 and 63 may be combined into a single comparison step 64 to determine whether to trigger an alert. Thus, there may be a combined step which assesses whether any combination of the predicted QOL score S (e.g. as generated in step 13), the difference ΔS (e.g. as generated in step 22) or the similarity value (e.g. as generated in step 62) meets one or more predetermined threshold values.

Thus, a single comparison step may determine whether one or more of the predicted QOL score, difference between a predicted QOL score and a previous QOL score and one or more similarity values (representing a similarity between a respective category of current health data and previous health data) meets one or more predetermined criteria.

For example, step 64 may determine to trigger an alert (i.e. move to step 15) when either: the predicted QOL score is less than a first predetermined threshold value and the difference ΔS is greater than a second predetermined threshold value; or the similarity value is less than a predetermined threshold value.

Any conceivable combination of criterions may be employed, and would be readily apparent to the skilled person.

Figure 7 illustrates a more detailed example for the step 62 of comparing previous health data to current health data and step 63 of determining whether a similarity value meets a predetermined criteria.

The current 11a and previous 61a health data both comprise a respective one or more categories or health measures. Each category represents a different measure of a subject's health (such as symptom information) or health-influencing factor (such as sleep, diet or exercise information). Thus, different portions of the health data relate to different categories or health measures.

In the illustrated example, the current health data comprise five categories 71b-71f (e.g. symptom occurrence, time spent asleep, exercise information, dietary information and emotional information) and the previous health data comprises a respective five categories 61b-61f. Each category of the current health data corresponds to a respective category of the previous health data and vice versa.

By way of example, the current health data 11a and previous health data may each comprise a first category 71b, 61b which indicates how a subject's symptoms (e.g. number of headaches per day) changes over time, and a second category 71c, 61c which details how a subject's amount of sleep changes over time.

In particular embodiments, health data may comprise one or more time series representative of a measure of health-related information changes over time. Thus, each category may be represented by a time series indicating how a measure of health-related information changes over a predetermined length of time (e.g. a month, week or day). Preferably, each time series of the current/previous health data is associated with a same predetermined length of time.

In step 62, respective categories of the current health data and the previous health data are compared to one another to generate respective similarity values.

Thus, a first category 71b of the current health data 11a is compared to a first category 62b of the previous health data to generate a first similarity value. Similarly, a second category 71c of the current health data 11a is compared to a second category 62c of the previous health data to generate a second similarity value.

Where each category is represented by a time series, the step 62 may comprise comparing a first time series (of the current health data) to a second time series (of the previous health data). As there may be a plurality of corresponding categories, step 62 may comprise comparing each of a plurality of first time series to a respective second time series.

In particular, step 62 may comprise calculating a time series correlation between a first time series (of a category of the current health data) and a second time series (of a corresponding category of the previous health data) to generate a similarity value. This time series correlation may be repeated for each respective time series (of respective categories) of the current and previous health data.

In step 64, each similarity value is compared to a predetermined threshold value to determine whether to trigger an alert (in step 15). For example, in response to any one of the similarity values being lower than the predetermined threshold value, an alert may be triggered.

The predetermined threshold value may vary for different categories. For example, where a category represents symptom information, the associated predetermined threshold value may be between 20-40% of the maximum possible similarity value (e.g. 30%). By way of another example, where a category represents a measure of the subject's sleep, the associated predetermined threshold value may be between 70-90% of the maximum possible similarity value (e.g. 80%). The value of the predetermined threshold value may depend upon the importance of a category in representing a condition of the patient.

Preferably, the alert triggering in step 15 indicates information about the category that has triggered the alert. For example, if a first similarity value, representing a similarity between the first category of the current health data and the corresponding first category of the previous health data, is below the predetermined threshold value, the alert triggering in step 15 may indicate that the first category of the health data has changed significantly.

In the illustrated example of Figure 7, it will be seen that the second through fifth categories (e.g. representing measures of sleeping, exercise, diet and mood) of the current health have not significantly changed compared to the previous health data. However, the first category (e.g. symptom information) has significantly changed. Thus, the step 15 may comprise triggering an alert indicating that a symptom of the subject has changed significantly.

In this way, an alert will be triggered which indicates abnormal changes to a subject's health-related information. This increases an effectiveness of the alerting method, as sudden changes in a subject's condition can be identified by monitoring sudden changes in signs/symptoms (or other health measures) of the subject.

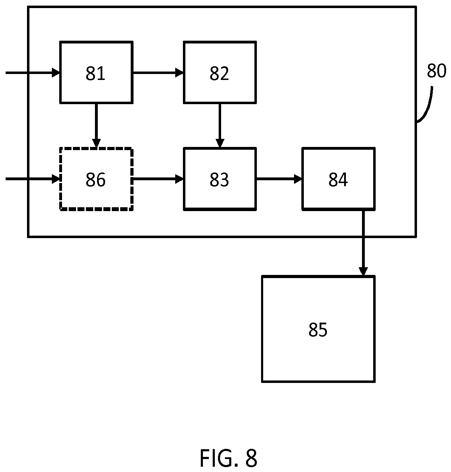

Figure 8 illustrates a system 80 for triggering an alert for a subject according to an embodiment of the invention. The system 80 comprises an obtaining unit 81, a predicting unit 82, a determining unit 83 and a triggering unit 84.

The obtaining unit 81 is adapted to obtain current health data of the subject; and obtain a prediction model for predicting a quality of life, QOL, score of the subject based on the current health data of the subject, wherein the prediction model is generated by performing a machine learning algorithm using a dataset formed of a plurality of data records, wherein each data record of the dataset comprises health data and an associated QOL score.

The obtaining unit 81 may be adapted to generate the prediction model, for example, by performing a machine learning algorithm using a dataset formed of a plurality of data records, wherein each data record of the dataset comprises health data and an associated QOL score. Other suitable methods have been previously described.

The obtaining unit 81 may be adapted to perform a natural language processing procedure on health notes of the subject in order to generate (and thereby obtain) current health data of the subject.

The predicting unit 82 is adapted to predict, using the prediction model, a QOL score of the subject based on the current health data of the subject. In other words, the predicting unit may receive, as input, at least health data of the subject in order to predict their QOL score.

The determining unit 83 is adapted to determine whether the predicted QOL score meets at least one predetermined criterion. This may comprise comparing the predicted QOL score to one or more predetermined values to determine whether one or more predetermined criteria are met. In other examples, as previously explained, this may comprise calculating a difference between the predicted QOL score and a previous QOL, and comparing this difference to one or more predetermined values to determine whether one or more predetermined criteria are met.

The triggering unit 84 is adapted to, in response to the predicted QOL score meeting the at least one predetermined criterion, trigger an alert. In particular, the triggering unit may generate an alert via an alerting mechanism 85, which may be external to the system 80 or, preferably, formed as part of the system 80.

In some embodiments, the system 80 further comprises a health data comparator 86 adapted to generate at least one similarity score indicative of a similarity between current health data and previous health data of the subject.

In some examples, as previously described, the current health data and the previous health data are both divisible into a same number of respective categories or time data series, e.g. each associated with a different measure of health-related information of the subject. The health data comparator may be adapted to generate a respective similarity score for each category, indicating of a similarity between that category of the current health data and a corresponding category of the previous health data.

In particular, the health data comparator 86 is adapted to obtain previous health data (e.g. from an external database) of the subject and the current health data from the obtaining unit 81. In other examples, the previous health data is obtained by the obtaining unit 81 and passed to the health data comparator.

The determining unit 83 may be adapted to determine whether the (at least one) similarity score and the predicted QOL score meet one or more criteria or combination of criteria. In response to any of the criteria, or combination of criteria, being met, the triggering unit 84 is adapted to trigger an alert.

The determining unit 83 may therefore be a combination module, which combines information from the predicting unit 82 and the health data comparator 86 to determine whether to trigger an alert. In this way, detailed changes of a subject's health condition can be detected.

The system 80 may be appropriately modified (e.g. modify existing units or include additional modules or units) for performing the steps of any previously described method.

The alert triggered by the system and method of the invention may be presented on a display, provided as an audible output or provided as a message to a remote device. When using a display, a visual alert is displayed when health data of a subject is processed and it is determined that one or more criteria has been met, which indicate a poor subject condition.

For example, a first type of visual alert may be generated if a predicted QOL score for health data gathered during a period meets predetermined criteria (e.g. is below a predetermined value).

In another example, a second type of visual alert may be generated for a period, if a similarity value (between a first category of current health data associated with the period and previous health data associated with a period immediately preceding the second period) meets predetermined criteria (e.g. is below a predetermined value).

The visual alert may differ based on the cause of the alert. For example, the first type of visual alert may be different to the second type of visual alert.

More detailed information may be provided, for example, in response to a user clicking on the visual alert - such as providing details about what triggered the alert.

As discussed above, embodiments make use of a system for triggering an alert. The system can be implemented in numerous ways, with software and/or hardware, to perform the various functions required. A processor is one example of a system which employs one or more microprocessors that may be programmed using software (e.g., microcode) to perform the required functions. A system may however be implemented with or without employing a processor, and also may be implemented as a combination of dedicated hardware to perform some functions and a processor (e.g., one or more programmed microprocessors and associated circuitry) to perform other functions.

Examples of system components that may be employed in various embodiments of the present disclosure include, but are not limited to, conventional microprocessors, application specific integrated circuits (ASICs), and field-programmable gate arrays (FPGAs).

In various implementations, a processor or system may be associated with one or more storage media such as volatile and non-volatile computer memory such as RAM, PROM, EPROM, and EEPROM. The storage media may be encoded with one or more programs that, when executed on one or more processors and/or systems, perform the required functions. Various storage media may be fixed within a processor or system or may be transportable, such that the one or more programs stored thereon can be loaded into a processor or system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method (1) of triggering an alert for a subject, the method comprising:
obtaining (11) current health data (11 a) of the subject;
obtaining (12) a prediction model (12a) for predicting a quality of life, QOL, score of the subject based on the current health data of the subject, wherein the prediction model is generated by performing a machine learning algorithm using a dataset formed of a plurality of data records, wherein each data record of the dataset comprises health data and an associated QOL score;
predicting (13), using the prediction model, a QOL score (S) of the subject based on the current health data of the subject;
determining (14) whether the predicted QOL score meets at least one predetermined criterion, and
in response to the predicted QOL score meeting the at least one predetermined criterion, triggering (15) an alert.

2. The method of claim 1, wherein the step (40) of obtaining the prediction model comprises generating (45) a prediction model (12a) by performing a machine learning algorithm on the dataset (41), wherein each data record of the dataset comprises health data (43a) and an associated QOL score (43b).

3. The method of claim 2, further comprising generating health data for each data record of the dataset by performing (42) a natural language processing procedure on health notes of a subject associated with a QOL score.

4. The method of any of claims 1 to 3, further comprising obtaining (12e) demographic information (12d) of the subject,
wherein the step of obtaining the prediction model comprises obtaining the prediction model based on at least the demographic information of the subject.

5. The method of any of claims 1 to 4, further comprising obtaining (12e) diagnosis and/or recommended treatment information (12d) of the subject,
wherein the step of obtaining the prediction model comprises obtaining the prediction model based on at least the diagnosis and/or recommended treatment information of the subject.

6. The method of any of claims 1 to 5, wherein the step of determining whether the predicted QOL score meets at least one predetermined criterion comprises determining (14, 33) whether the predicted QOL score is less than a first predetermined value.

7. The method of any of claims 1 to 6, wherein the step of determining whether the predicted QOL score meets the at least one predetermined criterion comprises determining (23, 33) whether a difference between the predicted QOL score (S) and a previous QOL score (ΔS) of the subject is greater than a second predetermined value.

8. The method of any of claims 1 to 7, wherein the current health data comprises at least one measure of a sign, symptom, lifestyle and/or emotion of the subject.

9. The method of any of claims 1 to 8, wherein the current health data (11a) comprises one or more categories (71b, 71c, 71d, 71e, 71f) of current health information of the subject, the method further comprising:
obtaining (61) previous health data (61a) comprising a respective one or more categories (61b, 61c, 61d, 61e, 61f) of previous health information of the subject;
comparing (62) each category of the current heath data to a respective category of the previous heath data to obtain a respective one or more similarity values indicative of a similarity between a category of the current health data and the respective category of the previous health data; and
in response to at least one of the one or more similarity values being lower than a predetermined value, triggering (15) an alert.

10. The method of claim 9, wherein:
each category (71b, 71c, 71d, 71e, 71f) of the current health data (11a) comprises a respective first time series representative of a change in current health information of the subject over time;
each category (61b, 61c, 61d, 61e, 61f) of the previous health data (61a) comprises a respective second time series representative of a change in previous health information of the subject over time,
and wherein the step (62) of comparing each category of the current health data to a respective category of the previous health data comprises comparing the first time series, of each category of the current health data, to the second time series, of a respective category of the previous health data, to obtain the similarity value.

11. The method of any of claims 1 to 10, wherein the step (11) of obtaining current health data of the subject comprises performing a natural language processing procedure on health notes of the subject to obtain the current health data.

12. The method of any of claims 1 to 11, wherein:
the current health data of the subject comprises health information gathered over the course of a time period of a predetermined length, and
the health data contained in a data record of the dataset comprises health information gathered over the course of a time period of the same predetermined length.

13. The method of claim 12, wherein the predetermined time interval is a month.

14. A computer program comprising code means for implementing the method of any one of claims 1 to 13 when said program is run on a computer.

15. A system (80) for triggering an alert for a subject, the system comprising:
an obtaining unit (81) adapted to:
obtain current health data of the subject; and
obtain a prediction model for predicting a quality of life, QOL, score of the subject based on the current health data of the subject, wherein the prediction model is generated by performing a machine learning algorithm using a dataset formed of a plurality of data records, wherein each data record of the dataset comprises health data and an associated QOL score;
a predicting unit (82) adapted to predict, using the prediction model, a QOL score of the subject based on the current health data of the subject;
a determining unit (83) adapted to determine whether the predicted QOL score meets at least one predetermined criterion, and
a triggering unit (84) adapted to, in response to the predicted QOL score meeting the at least one predetermined criterion, trigger an alert.
